Europäisches Patentamt

European Patent Office

Office européen des brevets

(11) Veröffentlichungsnummer: **0 408 077 B1**

(19)

(12) **EUROPÄISCHE PATENTSCHRIFT**

(45) Veröffentlichungstag der Patentschrift: **30.11.94**

(51) Int. Cl.⁵: **C12Q 1/68**, C07H 21/04

(21) Anmeldenummer: **90113485.8**

(22) Anmeldetag: **13.07.90**

(54) **Neisseria gonorrhoeae-spezifische Oligonuleotid-Sonden.**

(30) Priorität: **14.07.89 DE 3923341**

(43) Veröffentlichungstag der Anmeldung:
**16.01.91 Patentblatt 91/03**

(45) Bekanntmachung des Hinweises auf die
Patenterteilung:
**30.11.94 Patentblatt 94/48**

(84) Benannte Vertragsstaaten:
**AT BE CH DE DK ES FR GB GR IT LI LU NL SE**

(56) Entgegenhaltungen:
**EP-A- 0 237 737**
**EP-A- 0 337 896**
**WO-A-88/03957**

**JOURNAL OF GENERAL MICROBIOLOGY,
Band 135, Nr. 6, Juni 1989, SGM, GB; R.
ROSSAU et al., Seiten 1735-1745&NUM;**

(73) Patentinhaber: **BOEHRINGER MANNHEIM
GMBH**

**D-68298 Mannheim (DE)**

(72) Erfinder: **Wolff, Karin
Friedlandstrasse 23
D-8034 Germering (DE)**
Erfinder: **Stern, Anne, Dr.
Karwendelstrasse 10
D-8122 Penzberg (DE)**
Erfinder: **Buckel, Peter, Dr.
Eichenstrasse 19
D-8131 Bernried (DE)**
Erfinder: **Stackebrandt, Erko, Prof.
Sturenhagener Weg 5
D-2307 Dänischenhagen (DE)**

(74) Vertreter: **Huber, Bernhard, Dipl.-Chem. et al
Patentanwälte
H. Weickmann, Dr. K. Fincke
F.A. Weickmann, B. Huber
Dr. H. Liska, Dr. J. Prechtel, Dr. B. Böhm
Postfach 86 08
20
D-81635 München (DE)**

**Beschreibung**

Die Erfindung betrifft spezifische Nukleinsäure-Sonden und ein Verfahren zum Nachweis des Erregers Neisseria gonorrhoeae.

N. gonorrhoeae ist der Erreger der Gonorrhoe, der auch heute noch häufigsten meldepflichtigen Infektionskrankheit der Welt (World Health Statistics Annual (1979), Geneva, WHO). Beim Mann macht sich die genitale Infektion durch eitrige Entzündung und Schwellung der Urethramündung bemerkbar. Diese Symptome treten bei 90% der Infektionsfälle auf. Unbehandelt kann die Infektion aszendieren und nach einigen Wochen die Symptome einer Prostatitis auslösen. Bei der Frau treten dagegen bei 50 % der Infektionsfälle keine oder nur geringfügige Symptome auf. Die Infektion betrifft vor allem die Zervix, aber auch die Urethra. Bei 10 bis 15% der Frauen greift die Infektion auf die Eileiter über, was u.a. auch Sterilität zur Folge haben kann. In 1 bis 3% der Fälle bei Mann oder Frau kann eine systemische Invasion durch den Erreger stattfinden, wodurch Arthritis, Endocarditis und Peritonitis hervorgerufen werden können. Da die Infektionen häufig asymptomatisch verlaufen, wirken viele Träger unwissentlich an der Verbreitung der Krankheit mit (Davis et al., In:Microbiology; Harper International Edition).

Die Gattung Neisseria stellt eine Gruppe eng verwandter, gramnegativer Diplokokken dar, zu der sowohl pathogene wie auch apathogene Spezies gehören. Daher ist die Unterscheidung von N. gonorrhoeae von anderen nichtpathogenen Spezies, die die Schleimhäute des Menschen besiedeln, sehr wichtig für die nachfolgende Behandlung. Es besteht somit häufig die Gefahr einer Verwechslung, wenn die Diagnose auf Gonorrhoe ausschließlich auf mikroskopischem Weg erfolgt.

Der definitive diagnostische Nachweis von Neisseria gonorrhoeae setzt das Anlegen einer Kultur voraus. Hierbei treten allerdings häufig Probleme des Materialtransportes und der Anzucht auf, da die Gonokokken aufgrund ihrer autolytischen Enzymsysteme gegenüber Umwelteinflüssen wie Temperaturveränderung und Austrocknung außerordentlich empfindlich sind. Außerdem ist die Kultivierung langwierig, da die Inkubationszeit mindestens 20 Stunden beträgt. Hinzu kommt, daß sowohl die Anzucht wie auch die definitive Identifizierung des Erregers schwierig sein kann und deshalb weitgehend in mikrobiologischen Fachlaboratorien stattfinden muß. Die Differenzierung der angezüchteten Gonokokken geschieht durch Kohlehydratabbau, Oxidasereaktion, Antigennachweis durch Coagglutination und Fluoreszenz-Antikörper-Screening.

Zur Kultivierung von N. gonorrhoeae wird ein Selektivmedium verwendet, auf dem jedoch auch apathogene Neisseria-Spezies, wie z.B. N. lactamica wachsen können. Aus diesem Grund besteht die Gefahr einer Verwechslung, die nur durch exakte und aufwendige biochemische Differenzierung ausgeschlossen werden kann. Es wurde deshalb versucht, Diagnosetests zu entwickeln, die schnell und spezifisch den Nachweis von Neisseria gonorrhoeae erlauben.

In den letzten Jahren wurde zur Identifizierung pathogener Organismen die vom Gesichtspunkt der Schnelligkeit her attraktive Technik der Nukleinsäure-Hybridisierung entwickelt. Auch in Bezug auf Neisseria gonorrhoeae hat man versucht, Nukleinsäure-Sonden zur Diagnose heranzuziehen.

Diagnostisch einsetzbare Nukleinsäure-Sonden müssen zwei Kriterien erfüllen. Sie müssen spezifisch sein, d.h. die Sonde darf nur mit der Nukleinsäure des nachzuweisenden Erregers hybridisieren, um "falsch-positive" Testergebnisse ausschließen zu können. Sie müssen auch sensitiv sein, d.h. mit Hilfe der Nukleinsäure-Hybridisierung sollten auch wenige Erreger nachgewiesen werden können, um "falsch-negative" Testergebnisse während eines frühen Stadiums der Infektion auszuschließen.

Bereits vor einigen Jahren wurde die Möglichkeit erkannt, daß mit Hilfe von Nukleinsäure-Sonden, die komplementär zur ribosomalen RNA (rRNA) sind, spezifisch Organismen nachgewiesen werden können. Solche Sonden weisen den Vorteil einer hohen Sensitivität auf, da zwischen 1000 Kopien und 10.000 Kopien (der rRNA) pro Zelle vorliegen.

Die Methode zur Identifizierung von Organismen mit Hilfe solcher rRNA-spezifischer Proben wurde bereits mehrfach beschrieben (EP-B 0155359, WO 84/02721, EP-A 0076123).

Auch für Neisseria gonorrhoeae wurden Sonden, deren Sequenzen komplementär zu Teilbereichen der 16S rRNA sind, zum spezifischen Nachweis dieses Erregers eingesetzt. Die Sequenz der Sonden wurde dabei von drei Teilbereichen der 1544 Nukleotide langen 16S rRNA aus N. gonorrhoeae abgeleitet (Nukleotid-Position 125-150, 455-485, 980-1015). Die genannten Sonden wurden als Gemisch gegen die gesamte RNA, die aus verschiedenen Neisseria-Spezies (N. gonorrhoeae, N. meningitidis, N. cinerea, N. lactamica, N. mucosa und N. subflava) und Kingella kingae isoliert worden war, hybridisiert. Die Proben wurden als spezifisch für N. gonorrhoeae (EP-A 0272009) angesehen.

Dieses Sonden-Gemisch wurde nun mit einer Auswahl an verschiedensten Spezies (s.u.) überprüft. Es wurde dabei eine Hybridisierung der bekannten Sonden mit der Nukleinsäure von Neisseria denitrificans, einer apathogenen, aber mit Neisseria gonorrhoeae eng verwandten Spezies, nachgewiesen.

Aufgabe der vorliegenden Erfindung war es daher, Nachweis-Sonden für Neisseria gonorrhoeae bereitzustellen, welche gegenüber den bisher bekannten rRNA-Sonden eine erhöhte Spezifität besitzen und somit einen sicheren qualitativen wie auch quantitativen Nachweis der pathogenen Spezies ermöglichen.

Diese Aufgabe wird gelöst durch eine für Neisseria gonorrhoeae spezifische Nukleotid-Sonde, welche ausgewählt ist aus der Gruppe der Oligonukleotide RS2 und RS3. Ein Oligonukleotid der Gruppe RS2 oder RS3 ist dadurch gekennzeichnet, daß seine Sequenz oder ein Teil seiner Sequenz komplementär zu denjenigen Teilbereichen R2 und R3 der 16S rRNA von Neisseria gonorrhoeae ist, wie sie in Tabelle I aufgeführt sind.

## Tabelle I

| Region | Nukleotidposition auf 16S rRNA von N. gonorrhoeae | |
|---|---|---|
| R2 | 820 – 860 | ATGTCAATTA GCTGTTGGGC AACTTGATTG CTTGGTAGCG T |
| R3 | 65 – 100 | GGACGGCAGC ACAGGGAAGC TTGCTTCTCG GGTGGC |

Mit diesen Sonden gelingt ein spezifischer Nachweis von Neisseria gonorrhoeae. Es wird weder Hybridisierung mit nahe verwandten Neisseria-Spezies, wie N. denitrificans, noch mit anderen Spezies gefunden. Die hohe Spezifität dieser Sonden ist von großer Bedeutung, weil in einem Specimen (z.B. Abstrich) viele nichtpathogene Neisseria-Spezies vorkommen können, die Bestandteil der menschlichen Schleimhaut sind. Aus diesem Grund wurden auch andere Mikroorganismen, welche die menschliche Schleimhaut besiedeln, aber nicht Neisseria-Spezies sind, in das Screening auf Spezifität aufgenommen.

Besonders gute Ergebnisse bezüglich der Spezifität werden mit bestimmten Sonden erzielt.

In einer besonders bevorzugten Ausführungsform der vorliegenden Erfindung verwendet man als Neisseria gonorrhoeae spezifische Nukleinsäure-Sonden die Oligonukleotide RS2* oder/und RS3* mit 18 bzw. 28 Nukleotiden Länge, deren Sequenz aus Tabelle II ersichtlich ist.

Ebenfalls besonders bevorzugt ist es, wenn man zum Nachweis von N. gonorrhoe Nukleinsäure-Sonden verwendet, welche zum gesamten Bereich R2 bzw. R3 komplementär sind und mit RS2** bzw. RS3** bezeichnet werden. RS2** besitzt demnach eine Länge von 41 Nukleotiden und RS3** ist 36 Nukleotide lang.

Die erfindungsgemäßen Sonden sind mindestens 14 Nukleotide lang, und können an ihrem 5'- und/oder 3'-Ende weitere Nukleotide, vorzugsweise bis zu 10 Nukleotide, aufweisen.

Die zusätzlichen Nukleotide der Sonde können hierbei beliebige Nukleotide sein, bevorzugt sind jedoch diejenigen Nukleotide, die komplementär zu den sich auf der 16S rRNA am 5'- bzw. 3'-Ende befindenden Nukleotiden sind.

Die erfindungsgemäßen, für Neisseria gonorrhoeae spezifischen Nukleinsäure-Sonden können weiterhin in verschiedenen Formen vorliegen. So können sie als einzelsträngige Oligonukleotide, mit einem komplementären Oligonukleotid als doppelsträngige Oligonukleotid-Fragmente, oder assoziiert mit anderen Sequenzen, die keine Homologie zur DNA oder RNA aus Neisseria gonorrhoeae aufweisen, z.B. Klonierungsvektoren, vorliegen. Es können ebenso modifizierte oder nicht modifizierte Ribonukleotide wie Desoxyribonukleotide verwendet werden. Hierbei ergibt sich wiederum die Möglichkeit der Unterscheidung in Einzelstrang-Vektoren, wie z.B. M13 und Doppelstrang-Vektoren, wie z.B. die pBR322-Derivate. Weiterhin können die Sequenzen der Suchproben in Einzel-oder Doppelstrangform miteinander gekoppelt werden, so daß zwei oder mehr dieser Sequenzen z.B. in einem Vektor vorliegen. Bei der Verwendung von Doppelstrang-

3

formen wird vor der tatsächlichen Nachweisreaktion durch Denaturierung eine Auftrennung der Sonde in die Einzelstränge bewirkt.

In einer weiteren bevorzugten Ausführungsform der Erfindung sind die Nukleinsäure-Sonden markiert. Dies ist prinzipiell möglich aber alle an sich bekannten Arten der Markierung, nämlich den Einbau von radioaktiven Isotopen, oder aber den Einbau aber eine nicht-radioaktive Markierung, z.B. über den Einbau modifizierter Nukleotide und einem entsprechenden Detektionssystem.

Ein weiterer Gegenstand der Erfindung ist ein Verfahren zum Nachweis von N. gonorrhoae unter Verwendung mindestens einer hybridisierenden Sonde bei Hybridisierungsbedingungen und Nachweis der Hybridbildung, das dadurch gekennzeichnet ist, daß man zum spezifischen Nachweis eine der erfindungsgemäßen Oligonukleotidsonden verwendet.

Die erfindungsgemäßen Oligonukleotidsonden hybridisieren sowohl mit der rRNA als auch mit den entsprechenden rRNA-Genen auf dem bakteriellen Genom. Es ist daher im erfindungsgemäßen Verfahren möglich, über beide Nukleotid-Typen die An- und Abwesenheit von N. gonorrhoeae quantitativ und qualitativ zu bestimmen.

Der Nachweis aber eine Hybridisierung mit mindestens einer der erfindungsgemäßen Sonden kann nach an sich bekannten Methoden zum Nachweis von Nukleinsäuren über Hybridisierung durchgeführt werden. Die DNA-DNA-Hybridisierung wurde erstmals von Southern in J.Mol.Biol. 98 (1975) 503 beschrieben und in der Folge weiterentwickelt. Alle geeigneten Nukleinsäure-Hybridisierungsansätze können Verwendung finden, wie z.B. Festphasen-Hybridisierung, Hybridisierung in Lösung, Sandwich-Hybridisierung, Zwei-Komponenten-Hybridisierung. Der Nachweis erfolgt dann aber entweder eine radioaktive oder nichtradioaktive Markierung der Sonde.

Erfindungsgemäß gelingt es durch Verwendung der genannten Nukleinsäure-Sonden, einen schnellen und sicheren Nachweis des Vorhandenseins von N. gonorrhoeae in Specimen und damit einer Infektion des Patienten zu führen. Hierzu ist das Anlegen einer Kultur nicht erforderlich.

Das folgende Beispiel soll die Erfindung erläutern.

## Beispiel 1

Mit Hilfe einer Slot-Blot-Apparatur wurde die chromosomale DNA der folgenden Spezies jeweils auf ein Slot der Apparatur auf einen Nitrocellulosefilter aufgebracht:
- pathogene Neisseria-Spezies:
  Neisseria gonorrhoeae:        4 unterschiedliche Isolate,
  Neisseria meningitidis:        4 unterschiedliche Isolate,
- apathogene Neisseria-Spezies:
  N. lactamica:                    3 unterschiedliche Isolate,
  N. mucosa:                       3 unterschiedliche Isolate,
  N. subflava, N. perflava, N. sicca:    2 unterschiedliche Isolate,
  N. elongata, N. cinerea:         2 unterschiedliche Isolate,
  N. flava:                        2 unterschiedliche Isolate, N. denitrificans.
- Non-Neisseria-Spezies: Haemophilus influenzae, Haemophilus parainfluenzae, Streptococcus salivarius, Streptococcus mutans, Streptococcus agalactiae, Staphylococcus aureus, Staphylococcus epidermidis, Staphylococcus saprophyticus, Escherichia coli, Kingella kingae.

Die Präparation der bakteriellen chromosomalen DNAs erfolgte nach dem von Stern et al., Cell 37 (1984), Seite 447 beschriebenen Protokoll.

Die Filter wurden hierzu in die Apparatur eingespannt, ein Vakuum angelegt und die einzelnen Auftragspunkte mit 200 $\mu$l 2x Puffer angefeuchtet (2x Puffer: 2 mol/l NaCl, 50 mmol/l Tris-HCl, pH 7,5 und 1 mmol/l EDTA). Die DNA wurde mit 50 $\mu$l 50 mmol/l Tris-HCl, pH 7,5 und 5 mmol/l EDTA versetzt und zur Denaturierung 3 Minuten gekocht. Anschließend wurde der Ansatz sofort auf Eis überführt, 50 $\mu$l 2xPuffer zugegeben und diese Lösung in die Slots pipettiert. Die Slots wurden mit 100 $\mu$l 1xPuffer (2xPuffer 1:1 mit Wasser verdünnt) nachgespült, der Filter der Apparatur entnommen und im Vakuum getrocknet. Die Hybridisierung erfolgte im wesentlichen nach den beschriebenen Methoden (Southern, J. Mol. Biol. 98 (1975), Seite 503).

Zur Feststellung der Spezies-Spezifität wurden die synthetisierten Oligonukleotide gegen die filtergebundene chromosomale DNA aus den verschiedenen Bakterien-Spezies hybridisiert. Hierzu wurden die Nitrocellulosefilter zwei Stunden in 1xVHP vorhybridisiert (2xVHP: 0,1 % Rinderserumalbumin, 0,1 % Ficoll 400 000, 0,1 % Polyvinylpropylidon, 1 % Glycerin, 1,8 mol/l NaCl, 50 mmol/l $Na_2HPO_4$ und 10 mg/l Heringssperma-DNA). Die im VHP enthaltenen Heringssperma-DNA war zuvor durch Erhitzen auf 80 °C denaturiert worden.

Die Hybridisierung erfolgte in einem Hybridisierungsofen bei 42°C. Der VHP wurde entfernt und durch die $^{32}$P-markierte Oligonukleotid-Probe in Hybridisierungspuffer (HP) ersetzt (HP: 1xVHP und Zugabe von 0 bis 30 % Formamid, je nach GC-Gehalt der Sonde). Der HP wurde vor Verwendung auf 80°C erhitzt. Nach einer Hybridisierungszeit von mindestens 6 Stunden wurden die Filter in Waschpuffer (WP: 0,36 mol/l NaCl, 10 mmol/l $Na_2HPO_4$, 0,05 % SDS) zunächst zweimal bei Raumtemperatur und dann zweimal bei 42°C gewaschen. Die Waschtemperatur wurde dann je nach GC-Gehalt der Probe bis auf maximal 68°C erhöht. Die tatsächlichen maximalen Waschtemperaturen sowie der Formamidgehalt sind auch in Tabelle II dargestellt. Diese Tabelle zeigt außerdem die Sequenzen der verwendeten Nukleinsäure-Sonden RS2* und RS3*.

## Tabelle II

| Bezeich- nung der Sonde | Sequenz der Sonde 5'->3' | Form- amid- gehalt des HP (%) | Wasch- tempera- tur (°C) |
|---|---|---|---|
| RS2* | TACCAAGCAATCAAGTTG | 0 | 50 |
| RS3* | CCACCCGAGAAGCAAGCTTCCCTGTGCT | 30 | 68 |

Die Filter wurden gegen einen Röntgenfilm exponiert.

In Tabelle III sind die Ergebnisse der Hybridisierung der verschiedenen Nukleinsäure-Sonden mit pathogenen, nichtpathogenen Neisseria-Spezies und Nicht-Neisseria-Spezies gezeigt.

5

Tabelle III

| Species[1] / Stamm | Isolat Nr. | Oligonukleotide RS2* | RS3* |
|---|---|---|---|
| Neisseria gonorrhoeae | 74 | + | + |
| " " | 514 | + | + |
| " " | r2 | + | + |
| " " | R16 | + | + |
| Neisseria meningitidis | B | - | - |
| " " | D | - | - |
| " " | 2-5 | - | - |
| " " | Z | - | - |
| Neisseria lactamica | 1855 | - | - |
| " " | 2879 | - | - |
| " " | 3272 | - | - |
| Neisseria mucosa | 112 | - | - |
| " " | 114 | - | - |
| " " | 2888 | - | - |
| Neisseria subflava | 124 | - | - |
| Neisseria perflava | 120 | - | - |
| Neisseria sicca | 2844 | - | - |
| " " | 118 | - | - |
| Neisseria elongata | 129 | - | - |
| Neisseria cinerea | 126 | - | - |
| " " | 2199 | - | - |
| Neisseria flava | 122 | - | - |
| " " | 123 | - | - |
| Neisseria denitrificans | 2950 | - | - |
| Haemophilus influenzae | 2214-1 | - | - |
| Haemophilus parainfluenzae | 1207 | - | - |
| Streptococcus salivarius | DSM 20067-1974 | - | - |
| Streptococcus mutans | ATCC 25175 | - | - |
| Streptococcus agalactiae | DSM 2104 | - | - |
| Staphylococcus aureus | 1472 | - | - |
| Staphylococcus epidermidis | ATCC 14990 | - | - |
| Staphylococcus saprophyticus | ATCC 15305 | - | - |
| Escherichia coli | 1424 | - | - |
| Kingella kingae | ATCC 23330 | - | - |

[1] klassifiziert nach Bergey's Manual of Systematic Bacteriology (1984) Krieg N.R., Holt J. G. (eds), Williams and Wilkins, Baltimore Seiten 288 bis 298.

**Patentansprüche**

**Patentansprüche für folgende Vertragsstaaten : AT, BE, CH, DE, DK, FR, GB, GR, IT, LI, LU, NL, SE**

1. Neisseria gonorrhoeae-spezifische Oligonukleotidsonde, **dadurch gekennzeichnet,** daß sie

   a) ein Oligonukleotid RS2* mit der Sequenz TACCAAGCAA TCAAGTTG,

   b) ein Oligonukleotid RS3* mit der Sequenz CCACCCGAGA AGCAAGCTTC CCTGTGCT,

   c) ein Oligonukleotid RS2** mit der Sequenz ACGCTACCAA GCAATCAAGT TGCCCAACAG CTAATTGACA T oder/und

   d) ein Oligonukleotid RS3** mit der Sequenz GCCACCCGAG AAGCAAGCTT CCCTGTGCTG CCGTCC

   ist.

2. Sonde nach Anspruch 1, **dadurch gekennzeichnet,** daß sie an ihrem 5'- und/oder 3'-Ende weitere Nukleotide aufweist.

3. Sonde nach Anspruch 2, **dadurch gekennzeichnet,** daß sie an ihrem 5'- und/oder 3'-Ende bis zu 10 weitere Nukleotide aufweist.

4. Sonde nach Anspruch 2 oder 3, **dadurch gekennzeichnet,** daß die weiteren Nukleotide denjenigen Nukleotiden entsprechen, die an dieser Stelle im natürlichen Gen, von dem sich die Sonden ableiten, vorhanden sind.

5. Sonde nach einem der Ansprüche 1 bis 4, **dadurch gekennzeichnet,** daß sie aus modifizierten oder nichtmodifizierten Ribonukleotiden oder Desoxyribonukleotiden besteht.

6. Sonde nach einem der Ansprüche 1 bis 5, **dadurch gekennzeichnet,** daß sie in einer oder mehreren Kopien auf einem einzel- oder doppelsträngigen Vektor enthalten ist.

7. Sonde nach einem der Ansprüche 1 bis 6, **dadurch gekennzeichnet,** daß sie in markierter Form vorliegt.

8. Verfahren zum Nachweis der pathogenen Neisseria-Spezies N. gonorrhoeae unter Anwendung einer hybridisierenden Sonde unter Hybridisierungsbedingungen und Nachweis der Hybridbildung, **dadurch gekennzeichnet,** daß man zum spezifischen Nachweis eine Sonde nach einem der Ansprüche 1 bis 7 verwendet.

9. Verfahren nach Anspruch 8, **dadurch gekennzeichnet,** daß man die Hybridbildung aber die Markierung der Sonde nachweist.

**Patentansprüche für folgenden Vertragsstaat : ES**

1. Verfahren zum Nachweis der pathogenen Neisseria-Spezies N. gonorrhoeae unter Anwendung einer hybridisierenden Sonde unter Hybridisierungsbedingungen und Nachweis der Hybridbildung,
   **dadurch gekennzeichnet,**
   daß man zum spezifischen Nachweis eine Sonde verwendet, die

   a) ein Oligonukleotid RS2* mit der Sequenz TACCAAGCAA TCAAGTTG,

   b) ein Oligonukleotid RS3* mit der Sequenz CCACCCGAGA AGCAAGCTTC CCTGTGCT,

   c) ein Oligonukleotid RS2** mit der Sequenz ACGCTACCAA GCAATCAAGT TGCCCAACAG CTAATTGACA T oder/und

   d) ein Oligonukleotid RS3** mit der Sequenz GCCACCCGAG AAGCAAGCTT CCCTGTGCTG CCGTCC

   ist.

2. Verfahren nach Anspruch 1,
   **dadurch gekennzeichnet,**
   daß die Sonde an ihrem 5'- und/oder 3'-Ende weitere Nukleotide aufweist.

EP 0 408 077 B1

3. Verfahren nach Anspruch 2,
**dadurch gekennzeichnet,**
daß die Sonde an ihrem 5'- und/oder 3'-Ende bis zu 10 weitere Nukleotide aufweist.

4. Verfahren nach Anspruch 2 oder 3,
**dadurch gekennzeichnet,**
daß die weiteren Nukleotide denjenigen Nukleotiden entsprechen, die an dieser Stelle im natürlichen Gen, von dem sich die Sonden ableiten, vorhanden sind.

5. Verfahren nach einem der Ansprüche 1 bis 4,
**dadurch gekennzeichnet,**
daß die Sonde aus modifizierten oder nicht-modifizierten Ribonukleotiden oder Desoxyribonukleotiden besteht.

6. Verfahren nach einem der Ansprüche 1 bis 5,
**dadurch gekennzeichnet,**
daß die Sonde in einer oder mehreren Kopien auf einem einzel- oder doppelsträngigen Vektor enthalten ist.

7. Verfahren nach einem der Ansprüche 1 bis 6,
**dadurch gekennzeichnet,**
daß die Sonde in markierter Form verwendet wird.

8. Verfahren nach einem der Ansprüche 1 bis 7,
**dadurch gekennzeichnet,**
daß man die Hybridbildung über die Markierung der Sonde nachweist.

**Claims**
**Claims for the following Contracting States : AT, BE, CH, DE, DK, FR, GB, GR, IT, LI, LU, NL, SE**

1. Neisseria gonorrhoeae-specific oligonucleotide probe,
**wherein**
it is
a) an oligonucleotide RS2* having the sequence TACCAAGCAA TCAAGTTG,
b) an oligonucleotide RS3* having the sequence CCACCCGAGA AGCAAGCTTC CCTGTGCT,
c) an oligonucleotide RS2** having the sequence ACGCTACCAA GCAATCAAGT TGCCCAACAG CTAATTGACA T or/and
d) an oligonucleotide RS3** having the sequence GCCACCCGAG AAGCAAGCTT CCCTGTGCTG CCGTCC.

2. Probe as claimed in claim 1, **wherein** it has further nucleotides at its 5' and/or 3' end.

3. Probe as claimed in claim 2, **wherein** it has up to 10 further nucleotides at its 5' and/or 3' end.

4. Probe as claimed in claim 2 or 3, **wherein** the further nucleotides correspond to those nucleotides which are present at this position in the natural gene from which the probes are derived.

5. Probe as claimed in one of the claims 1 to 4, **wherein** it consists of modified or unmodified ribonucleotides or deoxyribonucleotides.

6. Probe as claimed in one of the claims 1 to 5, **wherein** it is present in a single or double-stranded vector in one or more copies.

7. Probe as claimed in one of the claims 1 to 6, **wherein** it is present in a labelled form.

8. Method for the detection of the pathogenic Neisseria species N. gonorrhoeae using a hybridizing probe under hybridization conditions and detection of the hybrid formation, **wherein** a probe is used for the specific detection as claimed in one of the claims 1 to 7.

8

9. Method as claimed in claim 8, **wherein** the hybrid formation is detected via the labelling of the probe.

**Claims for the following Contracting State : ES**

1. Method for the detection of the pathogenic Neisseria species N. gonorrhoeae using a hybridizing probe under hybridization conditions and detection of the hybrid formation,
**wherein**
a probe is used for the specific detection which is
   a) an oligonucleotide RS2* having the sequence TACCAAGCAA TCAAGTTG,
   b) an oligonucleotide RS3* having the sequence CCACCCGAGA AGCAAGCTTC CCTGTGCT,
   c) an oligonucleotide RS2** having the sequence ACGCTACCAA GCAATCAAGT TGCCCAACAG CTAATTGACA T or/and
   d) an oligonucleotide RS3** having the sequence GCCACCCGAG AAGCAAGCTT CCCTGTGCTG CCGTCC.

2. Method as claimed in claim 1,
**wherein**
the probe has further nucleotides at its 5' and/or 3' end.

3. Method as claimed in claim 2,
**wherein**
the probe has up to 10 further nucleotides at its 5' and/or 3' end.

4. Method as claimed in claim 2 or 3,
**wherein**
the further nucleotides correspond to those nucleotides which are present at this position in the natural gene from which the probes are derived.

5. Method as claimed in one of the claims 1 to 4,
**wherein**
the probe consists of modified or unmodified ribonucleotides or deoxyribonucleotides.

6. Method as claimed in one of the claims 1 to 5,
**wherein**
the probe is present on a single or double-stranded vector in one or several copies.

7. Method as claimed in one of the claims 1 to 6,
**wherein**
the probe is present in a labelled form.

8. Method as claimed in one of the claims 1 to 7,
**wherein**
the hybrid formation is detected via the labelling of the probe.

**Revendications**
**Revendications pour les Etats contractants suivants : AT, BE, CH, DE, DK, FR, GB, GR, IT, LI, LU, NL, SE**

1. Sonde oligonucléotidique spécifique de Neisseria gonorrhoeae, caractérisée en ce qu'il s'agit
   a) d'un oligonucléotide RS2* de séquence TACCAAGCAA TCAAGTTG,
   b) d'un oligonucléotide RS3* de séquence CCACCCGAGA AGCAAGCTTC CCTGTGCT,
   c) d'un oligonucléotide RS2** de séquence ACGCTACCAA GCAATCAAGT TGCCCAACAG CTAATTGACA T et/ou
   d) d'un oligonucléotide RS3** de séquence GCCACCCGAG AAGCAAGCTT CCCTGTGCTG CCGTCC.

2. Sonde selon la revendication 1, caractérisée en ce qu'elle comporte des nucléotides supplémentaires à son extrémité 5' et/ou 3'.

**3.** Sonde selon la revendication 2, caractérisée en ce qu'elle comporte jusqu'à 10 nucléotides supplémentaires à son extrémité 5' et/ou 3'.

**4.** Sonde selon la revendication 2 ou 3, caractérisée en ce que les nucléotides supplémentaires correspondent aux nucléotides qui sont présents à cet endroit dans le gène naturel dont dérivent les sondes.

**5.** Sonde selon l'une des revendications 1 à 4, caractérisée en ce qu'elle consiste en ribonucléotides ou désoxyribonucléotides modifiés ou non modifiés.

**6.** Sonde selon l'une des revendications 1 à 5, caractérisée en ce qu'elle est contenue en une ou plusieurs copies sur un vecteur simple brin ou double brin.

**7.** Sonde selon l'une des revendications 1 à 6, caractérisée en ce qu'elle est présente sous forme marquée.

**8.** Procédé de mise en évidence de l'espèce de Neisseria pathogène N. gonorrhoeae par utilisation, dans des conditions d'hybridation, d'une sonde qui s'hybride et par mise en évidence de la formation d'hybrides, caractérisé en ce que l'on utilise pour la mise en évidence spécifique une sonde selon l'une des revendications 1 à 7.

**9.** Procédé selon la revendication 8, caractérisé en ce que l'on met en évidence la formation d'hybrides par le biais du marqueur de la sonde.

**Revendications pour l'Etat contractant suivant : ES**

**1.** Procédé de mise en évidence de l'espèce de Neisseria pathogène N. gonorrhoeae par utilisation, dans des conditions d'hybridation, d'une sonde qui s'hybride et par mise en évidence de la formation d'hybrides, caractérisé en ce que l'on utilise pour la mise en évidence spécifique une sonde qui est
  a) un oligonucléotide RS2* de séquence TACCAAGCAA TCAAGTTG,
  b) un oligonucléotide RS3* de séquence CCACCCGAGA AGCAAGCTTC CCTGTGCT,
  c) un oligonucléotide RS2** de séquence ACGCTACCAA GCAATCAAGT TGCCCAACAG CTAATTGACA T et/ou
  d) un oligonucléotide RS3** de séquence GCCACCCGAG AAGCAAGCTT CCCTGTGCTG CCGTCC.

**2.** Procédé selon la revendication 1, caractérisé en ce que la sonde comporte des nucléotides supplémentaires à son extrémité 5' et/ou 3'.

**3.** Procédé selon la revendication 2, caractérisé en ce que la sonde comporte jusqu'à 10 nucléotides supplémentaires à son extrémité 5' et/ou 3'.

**4.** Procédé selon la revendication 2 ou 3, caractérisé en ce que les nucléotides supplémentaires correspondent aux nucléotides qui sont présents à cet endroit dans le gène naturel dont dérivent les sondes.

**5.** Procédé selon l'une des revendications 1 à 4, caractérisé en ce que la sonde consiste en ribonucléotides ou désoxyribonucléotides modifiés ou non modifiés.

**6.** Procédé selon l'une des revendications 1 à 5, caractérisé en ce que la sonde est contenue en une ou plusieurs copies sur un vecteur simple brin ou double brin.

**7.** Procédé selon l'une des revendications 1 à 6, caractérisé en ce que la sonde est utilisée sous forme marquée.

**8.** Procédé selon l'une des revendications 1 à 7, caractérisé en ce que l'on met en évidence la formation d'hybrides par le biais du marqueur de la sonde.